# EUROPEAN PATENT APPLICATION

(11) **EP 4 382 053 A2**
(43) Date of publication of application: **12.06.2024**
(21) Application number: 23214435.2
(22) Date of filing: 05.12.2023
(51) Int. Cl.: A61B 17/00, A61M 11/02, A61M 25/00

(54) **THERAPEUTIC AGENT DELIVERY SYSTEMS HAVING IMPROVED POWDER CONSISTENCY**

(30) Priority: 05.12.2022 US 202263430134 P
(71) Applicant: Cook Medical Technologies, LLC, Bloomington, IN 47404 (US)
(72) Inventor: ROSCA, Inga, Limerick (IE); BENN, Niall, Limerick (IE); SLATTERY, David, Limerick (IE); LONG, Margaret, Limerick (IE)
(74) Representative: Leach, Sean Adam

(57) **Abstract**

The present embodiments provide systems suitable for delivering a therapeutic agent to a target site. In some embodiments, the system comprises a container for holding the therapeutic agent, a pressure source, and a catheter in fluid communication with the container. In one embodiment, the system further comprises a first inlet tube disposed at least partially within the container, and a second inlet tube disposed at least partially within the container at a location different than the first inlet tube. Pressurized fluid from the pressure source flows into the container via each of the first inlet tube and the second inlet tube. In other embodiments, a plate is disposed with the container, wherein the plate is able to move vertically within the container during delivery of the therapeutic agent.

## Description

### PRIORITY CLAIM

This invention claims the benefit of priority of U.S. Provisional Application Serial No. 63/430,134, entitled "Therapeutic Agent Delivery Systems Having Improved Powder Consistency," filed December 5, 2022, the disclosure of which is hereby incorporated by reference in its entirety.

### BACKGROUND

The present embodiments relate generally to medical devices, and more particularly, to systems and methods for delivering therapeutic agents to a target site.

There are several instances in which it may become desirable to introduce therapeutic agents into the human or animal body. For example, therapeutic drugs or bioactive materials may be introduced to achieve a biological effect. The biological effect may include an array of targeted results, such as inducing hemostasis, sealing perforations, reducing restenosis likelihood, or treating cancerous tumors or other diseases.

Many of such therapeutic agents are injected using an intravenous (IV) technique and via oral medicine. While such techniques permit the general introduction of medicine, in many instances it may be desirable to provide localized or targeted delivery of therapeutic agents, which may allow for the guided and precise delivery of agents to selected target sites. For example, localized delivery of therapeutic agents to a tumor may reduce the exposure of the therapeutic agents to normal, healthy tissues, which may reduce potentially harmful side effects.

Localized delivery of therapeutic agents has been performed using catheters and similar introducer devices. By way of example, a catheter may be advanced towards a target site within the patient, then the therapeutic agent may be injected through a lumen of the catheter to the target site. Typically, a syringe or similar device may be used to inject the therapeutic agent into the lumen of the catheter. However, such a delivery technique may result in a relatively weak stream of the injected therapeutic agent.

Moreover, it may be difficult or impossible to deliver therapeutic agents in a targeted manner in certain forms, such as a powder form, to a desired site. For example, if a therapeutic powder is held within a syringe or other container, it may not be easily delivered through a catheter to a target site in a localized manner that may also reduce potentially harmful side effects.

Further, there may be challenges associated with delivering consistent doses of powder from a reservoir of the container, such as dense packing of particles, clogging, haphazard or poor settling of the powder after preceding delivery bursts, and other instances where the powder may be difficult to settle or otherwise advance from the container.

### SUMMARY

The present embodiments provide systems and methods suitable for delivering a therapeutic agent to a target site. In various embodiments, the system comprises a container for holding the therapeutic agent, a pressure source in selective fluid communication with at least a portion of the container, and a catheter in fluid communication with the container and having a lumen sized for delivery of the therapeutic agent to a target site.

In one embodiment, the system comprises a first inlet tube disposed at least partially within the container, the first inlet tube having a first end that is upstream relative to a second end of the first inlet tube. Further, the system comprises a second inlet tube disposed at least partially within the container at a location different than the first inlet tube, the second inlet tube having a first end that is upstream relative to a second end of the second inlet tube. Pressurized fluid from the pressure source flows into the container via each of the first inlet tube and the second inlet tube.

In other embodiments, a system suitable for delivering a therapeutic agent to a target site comprises a plate disposed with the container, wherein the plate is able to move vertically within the container during delivery of the therapeutic agent.

Other systems, methods, features and advantages of the invention will be, or will become, apparent to one with skill in the art upon examination of the following figures and detailed description. It is intended that all such additional systems, methods, features and advantages be within the scope of the invention, and be encompassed by the following claims.

### BRIEF DESCRIPTION OF THE DRAWINGS

The invention can be better understood with reference to the following drawings and description. The components in the figures are not necessarily to scale, emphasis instead being placed upon illustrating the principles of the invention. Moreover, in the figures, like referenced numerals designate corresponding parts throughout the different views.
FIG. 1 is a perspective view of a system in accordance with one exemplary embodiment.
FIG. 2 is a schematic view of the system of FIG. 1 with a portion of a housing removed.
FIG. 3 is a side-sectional view of the container of the system of FIGS. 1-2.
FIG. 4A-4B are side schematic views of a system in accordance with an alternative embodiment, with select components omitted in FIG. 4A for illustrative purposes.
FIGS. 5A-5B are, respectively, a side schematic view of a system in accordance with a further alternative embodiment, and a perspective view of a plate suitable for use with the system of FIG. 5A.
FIGS. 6A-6B are side schematic views illustrating an alternative embodiment in first and second states, respectively.
FIGS. 7A-7B are side schematic views illustrating a further alternative embodiment in first and second states, respectively.

### DETAILED DESCRIPTION OF THE PREFERRED EMBODIMENTS

In the present application, the term "proximal" refers to a direction that is generally towards a physician during a medical procedure, while the term "distal" refers to a direction that is generally towards a target site within a patient's anatomy during a medical procedure.

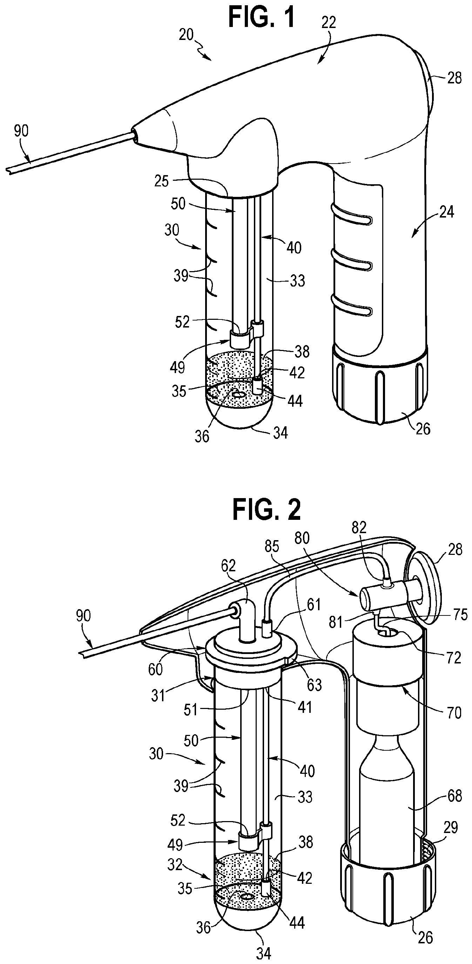

Referring now to FIGS. 1-3, a first embodiment of a system suitable for delivering one or more therapeutic agents is shown. In this embodiment, the system 20 comprises a container 30 that is configured to hold a therapeutic agent 38, and further comprises at least one pressure source 68 that is configured to be placed in selective fluid communication with at least a portion of the container 30, to deliver the therapeutic agent 3 8 through a catheter 90 to a target site within the patient, as explained more fully below.

The system 20 further comprises a housing 22, which is suitable for securely holding, engaging and/or covering the container 30, pressure source 68, catheter 90, and other components described below. Preferably, the housing 22 comprises an upright section 24 that may be grasped by a user and a section 25 for engaging the container 30. Actuators 26 and 28 may be engaged by a user and selectively operated to perform the functions described below.

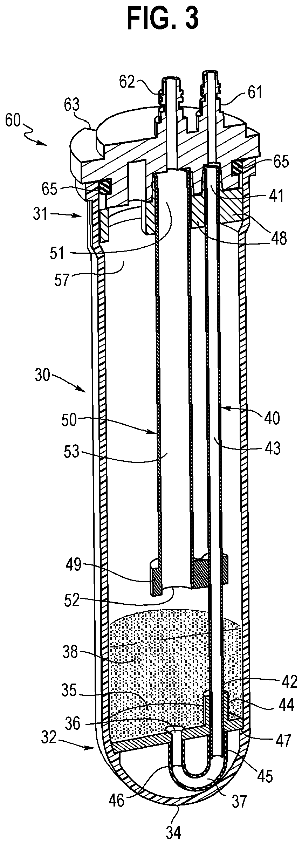

The container 30 may comprise any suitable size and shape for holding the therapeutic agent 38. In FIGS. 1-3, the container 30 comprises a generally tube-shaped configuration having a first region 31, a second region 32, and a reservoir 33 defined by an interior of the container 30. A platform 3 5 may be positioned within the container 30 above a curved end region 34, as best seen in FIG. 3.

The platform 35 preferably forms a substantially fluid tight seal with an inner surface of the container 30, thereby preventing the therapeutic agent 38 that is disposed in the reservoir 33 from reaching an inner portion of the curved end region 34, as shown in FIG. 3. In this embodiment, the platform 35 comprises an opening 36 though which fluid from the pressure source 68 is directed via a u-shaped tube 37 disposed within the curved end region 34, as shown in FIG. 3 and explained in further detail below.

The container 30 may further comprise an inlet tube 40, an outlet tube 50, and a cap 60, wherein the cap 60 is configured to be secured to the first region 31 of the container 30, as depicted in FIG. 3. The inlet tube 40 has first and second ends 41 and 42 with a lumen 43 extending therebetween, while the outlet tube 50 has first and second ends 51 and 52 with a lumen 53 extending therebetween. The first end 41 of the inlet tube 40 is placed in fluid communication with an inlet port 61 formed in the cap 60, while the first end 51 of the outlet tube 50 is placed in fluid communication with an outlet port 62 formed in the cap 60, as shown in FIG. 3.

The second end 42 of the inlet tube 40 extends towards the platform 35, and may be coupled to an adapter 44, which may be integral with the platform 35 or secured thereto. The adapter 44 places the second end 42 of the inlet tube 40 in fluid communication with a first end 45 of the u-shaped tube 37, which is disposed within the curved end region 34, as shown in FIG. 3. A second end 46 of the u-shaped tube 37 is in fluid communication with the opening 36 in the platform 35.

Accordingly, fluid passed through the inlet port 61 of the cap 60 is directed through the inlet tube 40, through the u-shaped tube 37, and into the reservoir 33 via the opening 36. Notably, the u-shaped tube 37 effectively changes the direction of the fluid flow by approximately 180 degrees, such that the fluid originally flows in a direction from the first region 31 of the container 30 towards the second region 32, and then from the second region 32 back towards the first region 31. In the embodiment of FIGS. 1-3, the first region 31 of the container 30 is disposed vertically above the second region 32 of the container 30 during use, however, it is possible to have different placements of the first and second regions 31 and 32 relative to one another, such that they are disposed at least partially horizontally adjacent to one another.

The second end 52 of the outlet tube 50 may terminate a predetermined distance above the platform 35, as shown in FIGS. 1-3. While the second end 52 is shown relatively close to the platform 35 in this embodiment, any suitable predetermined distance may be provided. For example, the outlet tube 50 may be shorter in length, e.g., about half of the length shown in FIGS. 1-3, and therefore, the second end 52 may be spaced apart further from the platform 35. In a presently preferred embodiment, the second end 52 of the outlet tube 50 is radially aligned with the opening 36 in the platform 35, as depicted in FIGS. 1-3. Accordingly, as will be explained further below, when fluid from the pressure source 68 is directed through the opening 36 in the platform 35, the fluid and the therapeutic agent 38 within the reservoir 33 may be directed through the outlet tube 50, through the outlet port 62, and towards a target site. Alternatively, the outlet tube 50 may be omitted and the therapeutic agent 38 may flow directly from the reservoir 33 into the outlet port 62. Other variations on the container 30 and the outlet port 62 may be found in U.S. Pat. No. 8,118,777 (hereafter "the '777 patent"), which is hereby incorporated by reference in its entirety.

The cap 60 may comprise any suitable configuration for sealingly engaging the first region 31 of the container 30. In one example, an O-ring 65 is held in place around a circumference of the cap 60 to hold the therapeutic agent 3 8 within the reservoir 3 3. Further, the cap 60 may comprise one or more flanges 63 that permit a secure, removable engagement with a complementary internal region of the section 25 of the housing 22. For example, by rotating the container 30, the flange 63 of the cap 60 may lock in place within the section 25.

The inlet and outlet tubes 40 and 50 may be held in place within the container 30 by one or more support members. In the example shown, a first support member 48 is secured around the inlet and outlet tubes 40 and 50 near their respective first ends 41 and 51, as shown in FIG. 3. The first support member 48 may be permanently secured around the inlet and outlet tubes 40 and 50, and may maintain a desired spacing between the tubes. Similarly, a second support member 49 may be secured around the inlet and outlet tubes 40 and 50 near their respective second ends 42 and 52, as shown in FIGS. 1-3. As will be apparent, greater or fewer support members may be provided to hold the inlet and outlet tubes 40 and 50 in a desired orientation within the container 30. For example, in one embodiment, the second support member 49 may be omitted and just the first support member 48 may be provided, or greater than two support members may be used.

In a loading technique, the inlet and outlet tubes 40 and 50 may be securely coupled to the first support member 48, the second support member 49, the platform 35 and the u-shaped tube 37. The platform 35 may be advanced towards the second region 32 of the empty container 30 until the platform rests on a step 47 above the curved end region 35 of the container 30, as shown in FIG. 3. In a next step, a desired quantity of the therapeutic agent 38 may be loaded through slits 57 formed adjacent to, or within, the first support member 48, as depicted in FIG. 3. Notably, the container 30 also may comprise measurement indicia 39, which allow a user to determine a quantity of the therapeutic agent 38 that is loaded within the reservoir 33 as measured, for example, from the top of the platform 35. With the therapeutic agent 38 loaded into the reservoir 33, the cap 60 may be securely coupled to the first region 31 of the container 30, and the container 30 then is securely coupled to the section 25 of the handle 22 as described above.

The pressure source 68 may comprise one or more components capable of producing or furnishing a fluid having a desired pressure. In one embodiment, the pressure source 68 may comprise a pressurized fluid, such as a liquid or gas. For example, as shown in FIG. 2, the pressure source 68 may comprise a pressurized fluid cartridge of a selected gas or liquid, such as carbon dioxide, nitrogen, or any other suitable gas or liquid that may be compatible with the human body. The pressurized fluid cartridge may contain the gas or liquid at a relatively high, first predetermined pressure, for example, around 1,800 psi inside of the cartridge. The pressure source 68 optionally may comprise one or more commercially available components. The pressure source 68 therefore may comprise original or retrofitted components capable of providing a fluid or gas at an original pressure.

The fluid may flow from the pressure source 68 through a pressure regulator, such as regulator valve 70 having a pressure outlet 72, as depicted in FIG. 2, which may reduce the pressure to a lower, second predetermined pressure. Examples of suitable second predetermined pressures are provided below.

The actuator 26 may be actuated to release the fluid from the pressure source 68. For example, a user may rotate the actuator 26, which translates into linear motion via a threaded engagement 29 between the actuator 26 and the housing 22, as shown in FIG. 2. When the linear advancement is imparted to the pressure source 68, the regulator valve 70 may pierce through a seal of the pressure cartridge to release the high pressure fluid. After the regulator valve 70 reduces the pressure, the fluid may flow from the pressure outlet 72 to an actuation valve 80 via tubing 75.

The actuation valve 80 comprises an inlet port 81 and an outlet port 82. The actuator 28, which may be in the form of a depressible button, may selectively engage the actuation valve 80 to selectively permit fluid to pass from the inlet port 81 to the outlet port 82. For example, the actuation valve 80 may comprise a piston having a bore formed therein that permits fluid flow towards the outlet port 82 when the actuator 28 engages the actuation valve 80. Fluid that flows through the outlet port 82 is directed into the inlet port 61 of the cap 60 via tubing 85, and subsequently is directed into the container 30, as explained above. It will be appreciated that any suitable coupling mechanisms may be employed to secure the various pieces of tubing to the various valves and ports.

The system 20 further may comprise one or more tube members for delivering the therapeutic agent 3 8 to a target site. For example, the tube member may comprise a catheter 90 having a proximal end that may be placed in fluid communication with the outlet port 62. The catheter 90 further comprises a distal end that may facilitate delivery of the therapeutic agent38 to a target site. The catheter 90 may comprise a flexible, tubular member that may be formed from one or more semi-rigid polymers. For example, the catheter may be manufactured from polyurethane, polyethylene, tetrafluoroethylene, polytetrafluoroethylene, fluorinated ethylenepropylene, nylon, PEBAX or the like. Further details of a suitable tube member are described in U.S. Pat. No. 12/435,574 (hereafter "the '574 patent"), the disclosure of which is hereby incorporated by reference in its entirety. As explained further in the ' 574 patent, a needle suitable for penetrating tissue may be coupled to the distal end of the catheter 90 to form a sharp, distal region configured to pierce through a portion of a patient's tissue, or through a lumen wall to perform a translumenal procedure.

In operation, the distal end of the catheter 90 may be positioned in relatively close proximity to the target site. The catheter 90 may be advanced to the target site using an open technique, a laparoscopic technique, an intraluminal technique, using a gastroenterology technique through the mouth, colon, or using any other suitable technique. The catheter 90 may comprise one or more markers configured to be visualized under fluoroscopy or other imaging techniques to facilitate location of the distal end of the catheter 90. If desired, the catheter 90 may be advanced through a working lumen of an endoscope.

When the catheter 90 is positioned at the desired target site, the pressure source 68 may be actuated by engaging the actuator 26. As noted above, the pressurized fluid may flow from the pressure source 68 through a regulator valve 70 and be brought to a desired pressure and rate. The fluid then flows through the tubing 75, and when the actuator 28 is selectively depressed, the fluid flows through the valve 80 and through the tubing 85 towards the container 30. The fluid is then directed through the inlet port 62, through the inlet tube 40 within the container 30, and through the u-shaped tube 37. At this point, the u-shaped tube effectively changes the direction of the fluid flow. Regulated fluid then flows through the opening 36 in the platform 35 and urges the therapeutic agent 38 through the outlet tube 50. The fluid and the therapeutic agent 38 then exit through the first end 51 of the outlet tube 50, through the outlet port 62 of the cap 60, and through the catheter 90, thereby delivering the therapeutic agent 38 to the target site at a desired pressure.

Optionally, a control mechanism may be coupled to the system 20 to variably permit fluid flow into and/or out of the container 30 at a desired time interval, for example, a predetermined quantity of fluid per second. In this manner, pressurized fluid may periodically flow into or out of the container 30 periodically to deliver the therapeutic agent 38 to a target site at a predetermined interval or otherwise periodic basis.

The system 20 may be used to deliver the therapeutic agent 38 in a wide range of procedures and the therapeutic agent38 may be chosen to perform a desired function upon ejection from the distal end of the catheter 90. Solely by way of example, and without limitation, the provision of the therapeutic agent 38 may be used for providing hemostasis, closing perforations, performing lithotripsy, treating tumors and cancers, treat renal dialysis fistulae stenosis, vascular graft stenosis, and the like. The therapeutic agent 38 can be delivered during procedures such as coronary artery angioplasty, renal artery angioplasty and carotid artery surgery, or may be used generally for treating various other cardiovascular, respiratory, gastroenterology or other conditions. The above-mentioned systems also may be used in transvaginal, umbilical, nasal, and bronchial/lung related applications.

For example, if used for purposes of hemostasis, thrombin, epinephrine, or a sclerosant may be provided to reduce localized bleeding. Similarly, if used for closing a perforation, a fibrin sealant may be delivered to a localized lesion. In addition to the hemostatic properties of the therapeutic agent 38, it should be noted that the relatively high pressure of the fluid and therapeutic agent, by itself, may act as a mechanical tamponade by providing a compressive force, thereby reducing the time needed to achieve hemostasis.

The therapeutic agent38 may be selected to perform one or more desired biological functions, for example, promoting the ingrowth of tissue from the interior wall of a body vessel, or alternatively, to mitigate or prevent undesired conditions in the vessel wall, such as restenosis. Many other types of therapeutic agents 38 may be used in conjunction with the system 20.

The therapeutic agent 38 may be delivered in any suitable form. For example, the therapeutic agent 38 may comprise a powder, liquid, gel, aerosol, or other substance. Advantageously, the pressure source 68 may facilitate delivery of the therapeutic agent 38 in any one of these forms.

The therapeutic agent 3 8 employed also may comprise an antithrombogenic bioactive agent, e.g., any bioactive agent that inhibits or prevents thrombus formation within a body vessel. Types of antithrombotic bioactive agents include anticoagulants, antiplatelets, and fibrinolytics. Anticoagulants are bioactive materials which act on any of the factors, cofactors, activated factors, or activated cofactors in the biochemical cascade and inhibit the synthesis of fibrin. Antiplatelet bioactive agents inhibit the adhesion, activation, and aggregation of platelets, which are key components of thrombi and play an important role in thrombosis. Fibrinolytic bioactive agents enhance the fibrinolytic cascade or otherwise aid in dissolution of a thrombus. Examples of antithrombotics include but are not limited to anticoagulants such as thrombin, Factor Xa, Factor VIIa and tissue factor inhibitors; antiplatelets such as glycoprotein IIb/IIIa, thromboxane A2, ADP-induced glycoprotein IIb/IIIa, and phosphodiesterase inhibitors; and fibrinolytics such as plasminogen activators, thrombin activatable fibrinolysis inhibitor (TAFI) inhibitors, and other enzymes which cleave fibrin.

Additionally, or alternatively, the therapeutic agent 38 may include thrombolytic agents used to dissolve blood clots that may adversely affect blood flow in body vessels. A thrombolytic agent is any therapeutic agent that either digests fibrin fibers directly or activates the natural mechanisms for doing so. Examples of commercial thrombolytics, with the corresponding active agent in parenthesis, include, but are not limited to, Abbokinase (urokinase), Abbokinase Open-Cath (urokinase), Activase (alteplase, recombinant), Eminase (anitstreplase), Retavase (reteplase, recombinant), and Streptase (streptokinase). Other commonly used names are anisoylated plasminogen-streptokinase activator complex; APSAC; tissue-type plasminogen activator (recombinant); t-PA; rt-PA. The therapeutic agent 3 8 may comprise coating-forming agents to protect or assist in healing of lesions and/or wounds.

In one example, the therapeutic agent 38 comprises a hemostasis powder manufactured by TraumaCure, Inc. of Bethesda, MD. However, while a few exemplary therapeutic agents 38 have been described, it will be apparent that numerous other suitable therapeutic agents may be used in conjunction with the system 20 and delivered through the catheter 90.

Advantageously, the system 20 permits localized delivery of a desired quantity of the therapeutic agent 3 8 at a desired, regulated pressure. Since the distal end of the catheter 90 may be placed in relatively close proximity to a target site, the system 20 provides significant advantages over therapeutic agents delivered orally or through an IV system and may reduce accumulation of the therapeutic agent 38 in healthy tissues, thereby reducing side effects. Moreover, the delivery of the therapeutic agent 38 to the target site is performed in a relatively fast manner due to the relatively high pressure of the fluid, thereby providing a prompt delivery to the target site compared to previous devices.

Further, if an optional needle is employed at the distal end of the catheter 90, as explained in the '574 patent, the system 20 advantageously may be used to both perforate tissue at or near a target site, then deliver the therapeutic agent 3 8 at a desired pressure in the manner described above. For example, the needle may comprise an endoscopic ultrasound (EUS) needle. Accordingly, in one exemplary technique, a sharpened tip of the needle may be capable of puncturing through an organ or a gastrointestinal wall or tissue, so that the therapeutic agent 3 8 may be delivered at a predetermined pressure in various bodily locations that may be otherwise difficult to access. One or more delivery vehicles, such as an endoscope or sheath, may be employed to deliver the catheter 90 to a target site, particularly if the distal end of the catheter 90 comprises the optional needle.

The therapeutic agent 3 8 must have a specific range of properties that make it suitable for delivery through the catheter 90, particularly when the catheter 90 is sized for delivery through a lumen of an endoscope. In particular, the mass of an individual particle of the therapeutic agent38 should be within a specific range. If a particle of the therapeutic agent 38 is too heavy, it will require too much pressure to travel the length of the catheter 90 and can result in clogging of the catheter 90. If the particle is too light, it will aerosolize within the patient's body, e.g., in the gastrointestinal space, instead of being propelled to a target site.

In addition to mass of an individual particle of the therapeutic agent 38, the size of the particle is important for ensuring proper delivery through the catheter 90. If the particle of the therapeutic agent 38 is too large in size, then it will be prone to clogging within the delivery catheter 90. If the particle is too small, it may have a higher likelihood of being aerosolized instead of being propelled to the target site.

In one embodiment, it has been found beneficial to have particles of the therapeutic agent 38 comprise a diameter in the range of about 1 micron to about 925 microns, and preferably in the range of about 45 microns to about 400 microns. Further, it has been found highly beneficial to have the particles of the therapeutic agent 38 comprise a mass in the range of about 0.0001 mg to about 0.5 mg, and preferably in the range of about 0.0001 mg to about 0.25 mg. It has been determined through multiple testing exercises that such ranges have criticality in terms of significantly reducing the likelihood of clogging of the catheter 90 during delivery, and also significantly reducing the likelihood of having the particles aerosolize during delivery, and therefore be properly delivered to a target site in the correct dose.

Particles of the therapeutic agent 38 may be ground, compacted and/or sieved to produce the desired particle size and mass. As used herein, particle mass is dependent on the density of the material and the volume of the particle. Further, regarding size, an assumption can be made that the particles are spheres, in which case the diameter ranges noted herein apply. However, it will be appreciated that other particle shapes exist, especially for crystalline materials. If the particle is substantially non-spherical, then similar micron ranges listed herein for spherical particles may apply, but instead of referring to diameter the value may refer to average or maximum width of the particle.

With regard to dimensions of the catheter 90, when used in endoscopic applications, it is clinically important to size the catheter 90 to be small enough to fit through a working lumen of the endoscope, yet be large enough to substantially avoid clogging when the therapeutic agent 3 8 is advanced through the catheter. In one embodiment, it has been found beneficial to have a ratio of catheter inner diameter to particle size diameter to be at least 4:1, and more preferably at least 7.5:1. The applicant has tested various embodiments, including a 400 micron particle being delivered through a 1.6 mm catheter (i.e., a 4:1 ratio) and determined that there is a risk of clogging. Accordingly, there is criticality in providing the ratio above 4:1, with any suitable size catheter that can be advanced through a lumen of an endoscope.

It should be noted that endoscopes are generally available with accessory channels up to 4.2 mm. Since a catheter inserted through this channel has a wall thickness of generally greater than 0.25 mm, the maximum projected inner diameter of the catheter for endoscopic delivery would be 3.7 mm. Based on a 4:1 ratio of catheter inner diameter to particle diameter, then the maximum acceptable particle diameter would be approximately 925 microns. Further, it is noted that spherical particles may be less susceptible to clogging than cuboid or flat particles. Accordingly, a ratio of closer to 4:1 may be acceptable for spherical particles, whereas a higher ratio (e.g., 7.5:1 or greater) is preferable for other particle shapes.

With regard to pressure, as noted above, the pressure source 68 may comprise a pressurized fluid cartridge of a selected gas or liquid, such as carbon dioxide, nitrogen, or any other suitable gas or liquid that may be compatible with the human body. The pressurized fluid cartridge may contain the gas or liquid at a relatively high, first predetermined pressure, for example, around 1,800 psi inside of the cartridge. The pressure source may be in a solid (dry ice), liquid or gas state. As further noted above, the fluid may flow from the pressure source 68 through a pressure regulator, such as regulator valve 70 having a pressure outlet 72, which may reduce the pressure to a lower, second predetermined pressure (referred to here as a "delivery system pressure"). In one embodiment, it has been found beneficial to have a delivery system pressure in the range of about 0.01 psi to about 100 psi, and preferably in the range of about 0.5 psi to about 75 psi. It has been determined through multiple testing exercises that such ranges have criticality in terms of providing appropriate force to propel the therapeutic agent 3 8 through the catheter 90, while significantly reducing the likelihood of clogging of the catheter 90 during delivery, and therefore properly delivering the therapeutic agent 3 8 to a target site in the correct dose. It should be noted that the applicant has also demonstrated delivery using a syringe filled with a powder and air that is manually compressed.

In view of Newton's Second Law (force equals mass times acceleration), acceleration of a particle of the therapeutic agent is dependent upon the particle mass and force applied to the particle. Therefore, a minimum force is necessary to overcome the force of gravity on the particles and to accelerate them to the desired velocity at the time at which they exit the distal end of the catheter 90. It is noted that increases in pressure of the pressure source 68 will deliver the therapeutic agent 38 more quickly, however, too high of a pressure can cause too high of a particle velocity and subsequently aerosolization.

There is a relationship between particle size, particle mass, and delivery velocity, which can be described by the drag equation: *F_{D}* = (1/2)(ρ)(*ν*²)(*C_{D}*)(*A*); and the gravitational force equation: *F_{G}* = (*m*)(*g*)*.* In these equations, ρ is the density of air (1.184 kg/m³), v is the velocity of the particles of the therapeutic agent 38, *C_{D}* is the drag coefficient (0.47 if the particles of the therapeutic agent 38 are assumed to be spherical), *A* is the cross-sectional area of a particle of the therapeutic agent 38, *m* is the mass of a particle of the therapeutic agent 38, and g is the acceleration due to gravity (9.81 m/s²).

Aerosolization occurs when the drag force exceeds the gravitational force on the particles of the therapeutic agent 38. Therefore, if the powder delivery velocity is too high relative to the mass of the particles, aerosolization can occur. The shape of the particles and size of the particles also should be factored into account, with more cubic shaped particles and larger particles requiring a lower delivery velocity so they do not aerosolize. In essence, for a given delivery system, there is a minimum particle mass at which aerosolization will occur.

In a preferred embodiment, the system of the present embodiments has a gravitational force *F_{G}* to drag force *F_{D}* ratio of preferably greater than 1:1. However, as the velocity of the particles of the therapeutic agent 38 rapidly decreases with drag force, systems with gravitational force *F_{G}* to drag force *F_{D}* ratios as small as 0.001:1 will clear within less than a minute.

Further details of preferred parameters for successfully delivering powder particles to a target site using a pressure source and catheter are described in U.S. Pat. No. 9,867,931 (hereafter "the '931 patent"), which is hereby incorporated by reference in its entirety.

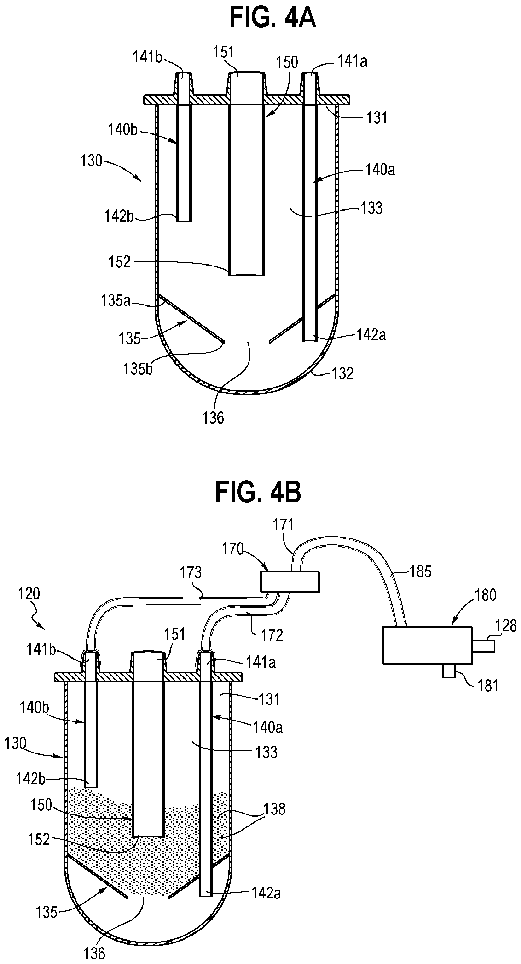

Referring now to FIGS. 4A-7B, various alternative systems are shown and described, compared to the system of FIGS. 1-3. The systems of FIGS. 4A-7B have design features that may be particularly well-suited for preventing powder clogging in the catheter 90, or may otherwise provide a more consistent delivery of powder to the target site, while overcoming challenges of the powder failing to flow in a smooth and predictable manner.

The embodiments of FIGS. 4A-7B are similar in certain respects to the system 20 of FIGS. 1-3, with pertinent differences noted below. For ease of reference, certain parts in FIGS. 4A-7B are identified by like reference numbers to similar parts explained in detail in FIGS. 1-3 above; for example, container 130 with regions 131 and 132 is similar to container 30 with regions 31 and 32, respectively, while platform 135 is similar to platform 35, outlet tube 150 is similar to outlet tube 50, and so forth for additional components.

Referring now to FIGS. 4A-4B, select components of a system 120 for delivering a therapeutic agent, particularly a powder, to a target site are shown and described. It is noted that the catheter 90 and other select parts are omitted in the depiction of the system 120 in FIGS. 4A-4B, but preferably are provided according to the catheter 90 and other parts discussed in FIGS. 1-3, with key distinctions noted below.

The system 120 of FIGS. 4A-4B comprises a container 130 having an alternative design compared to the container 30 of FIGS. 1-3. Notably, the container 130 comprises a first inlet tube 140a and a second inlet tube 140b, each of which delivers pressurized fluid into a reservoir 133 of the container 130.

As shown in FIGS. 4A-4B, the first inlet tube 140a has first and second ends 141a and 142a with a lumen extending therebetween, while the second inlet tube 140b has first and second ends 141b and 142b with a lumen extending therebetween. The first inlet tube 140a and the second inlet tube 140b are spaced-apart relative to each other in at least two directions.

In one direction, the first and second inlet tubes 140a and 140b are spaced-apart in a circumferential direction relative to each other. In particular, the first inlet tube 140a is depicted on the right side of the container 130 (from the viewpoint of FIGS. 4A-4B), while the second inlet tube 140b is depicted on the left side of the container 130. In one example, the spacing is approximately 180 degrees, although it will be appreciated that the spacing may range from about 60 to about 300 degrees, so long as the objectives described below can be achieved.

In a second direction, the second (or lower) ends of the first and second inlet tubes 140a and 140b are spaced-apart vertically relative to each other. In particular, the second end 142a of the first inlet tube 140a is depicted as vertically beneath the platform 135, while the second end 142b of the second inlet tube 140b is depicted as vertically above the platform 135, as shown in FIGS. 4A-4B. It should be noted that the second end 142b may be disposed above the therapeutic agent 138, or may be submerged within the therapeutic agent 138.

In this example, as shown in FIG. 4B, a flow splitter 170 may be provided upstream relative to the container 130, and downstream relative to an actuation valve 180. The actuation valve 180 may be similar to the actuation valve 80 described above, and comprises an inlet port 181 and outlet tubing 185. An actuator 128, which may be in the form of a depressible button, may engage the actuation valve 180 to selectively permit fluid to pass from the inlet port 181 to the outlet tubing 185. For example, the actuation valve 180 may comprise a piston having a bore formed therein that permits fluid flow towards the outlet tubing 185 when the actuator 128 engages the actuation valve 180, as described above in FIGS. 1-3. However, in the example of FIG. 4B, fluid that flows through the outlet tubing 185 is directed into an inlet port 171 of the flow splitter 170.

The flow splitter 170 is coupled to first and second outlet conduits 172 and 173, such as ports and associated tubing. The first outlet conduit 172 of the flow splitter 170 is in fluid communication with the first end 141a of the first inlet tube 140a, while the second outlet conduit 173 of the flow splitter 170 is in fluid communication with the first end 141b of the second inlet tube 140b, as depicted in FIG. 4B. In this manner, pressurized fluid from a single pressure source, such as source 68 of FIGS. 1-2, is directed through the valve 180, then through the flow splitter 170, and is ultimately routed to each of the first and second inlet tubes 140a and 140b simultaneously. It should be noted that, in alternative embodiments, the flow splitter 170 may be positioned within the valve 180, such that the first and second outlet conduits 172 and 173 effectively replace the outlet tubing 185 and a stand-alone flow splitter 170 is omitted.

During operation, the pressurized fluid that is routed through the first inlet tube 140a flows beneath the platform 135, in a first direction to a second direction, and then is re-directed through an opening 136 in the platform 135 and into the second end 152 of the outlet tube 150. As described above with respect to FIGS. 1-3, a combination of the pressurized fluid and the therapeutic agent then flows from the second end 152 towards the first end 151 of the outlet tube 150, and towards the catheter 90 for delivery to the target site.

During this process, the pressurized fluid that is routed through the second inlet tube 140b exits above the platform 135, and this fluid routing helps "shake" particles of the therapeutic agent 138 free on a regular basis throughout the agent's delivery, which may reduce clogging in the container 130. In other words, the particles of the therapeutic agent 138 are less likely to settle in a compressed, packed or otherwise static manner relative to one another, similar to cooking flour that has not been agitated for a period of time. Instead, the fluid routed to the second inlet tube 140b provides a gentle agitation to keep the particles looser (compared to no agitation at all) and therefore less likely to become static and clog.

As a further advantage, the provision of the two inlet tubes 140a and 140b in this arrangement may promote a more consistent dose of powder, particularly when a user is periodically pressing the button 128 to switch between "on" and "off" states of powder delivery. Specifically, pressurized fluid from the second inlet tube 140b may reduce aeration time between user-actuated sprays of the powder, as the flow shaking and agitation provided by the second inlet tube 140b helps the powder settle in a faster and more consistent manner.

It is noted that some powders have high "air retention" properties, and for such powders, the settling time can run into the hours. The agitation or vibration provided by the pressurized fluid from the second inlet tube 140b can speed up the settling time.

In one embodiment, the flow splitter 170 may evenly distribute flow to the first and second outlet conduits 172 and 173. However, in alternative embodiments, the flow splitter 170 may distribute flow unequally, for example, by allowing a higher pressure to pass through the first outlet conduit 172 and into the first inlet tube 140a. In one example, the flow splitter 170 may be configured such that the pressure of the fluid moving through the first outlet conduit 172 is about 1.2 to about 4.0 times greater than the fluid flowing through the second outlet conduit 173, and more preferably, about 2.0 to about 3.0 greater. In this manner, the second inlet tube 140b provides slightly less pressure to shake the powder above the platform 135 in a relatively gentle manner (without causing too much disruption that can cause dilation of the therapeutic agent 138 instead of even settling above the platform 135), while the first inlet tube 140a provides slightly higher pressure as the primary driver to move the therapeutic agent 138 the requisite speed through the outlet tube 150 and the catheter 90.

Notably, in FIGS. 4A-4B, the platform 135 is shown having a tapered or angled design, where an outer region 135a is vertically above an inner region 135b that is closest to the opening 136. This design may have advantages in terms of routing powder towards the opening 136 using gravity of the taper in the platform; however, it will be appreciated that the generally flat platform 35 depicted in FIGS. 1-3 may be used in the design of FIGS. 4A-4B.

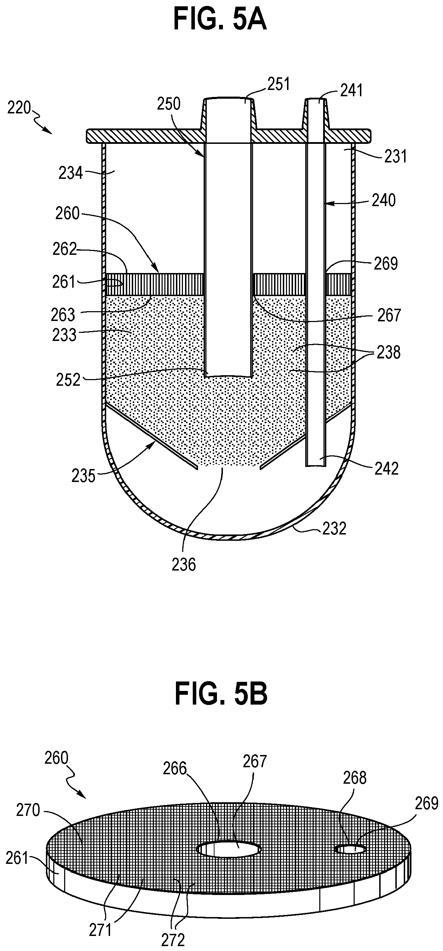

Referring now to FIGS. 5A-5B, a further alternative system 220 comprises a container 230 which is similar to the container 30 of FIGS. 1-3, but which comprises a plate 260 disposed within a reservoir 233 that holds a therapeutic agent 238.

In one embodiment, the plate 260 comprises an upper surface 262, a lower surface 263, and an outer perimeter 261 having a diameter that is substantially the same, or slightly less, than an inner diameter of the container 230. In this manner, the outer perimeter 261 of the plate 260 is substantially flush with the interior of the container 230, as depicted in FIG. 5A, and the plate 260 has an ability to move vertically within the container 230 (i.e., in a direction from a first region 231 towards a second region 232, and vice versa).

In one embodiment, the plate 260 is centered around the outlet tube 250, as shown in FIG. 5A. The plate 260 may comprise a first ring 266 having a bore 267 (which may be centrally-located if the plate is circular), through which the outlet tube 250 extends, and further comprises a second ring 268 having a bore 269 through which the inlet tube 240 extends. The first and second bores 267 and 269 are dimensioned with a small clearance relative to the outlet tube 250 and the inlet tube 240, respectively, such that vertical movement of the plate 260 is enabled.

In one example, the plate 260 may be porous by providing a mesh or gauze 270, which may comprise metal or plastic. The mesh 270 may comprise multiple strands 271 extending in a first direction, and multiple strands 272 extending in a second direction, where the first and second directions are substantially perpendicular to one another. The mesh 270 may comprise a generally circular shape with an exterior perimeter that approximates the outer perimeter 261 of the plate 260, and the mesh 270 may omit material in the region of the first and second bores 267 and 269, as shown in FIG. 5B, thereby allowing passage of the outlet tube 250 and the inlet tube 240, respectively.

The size of the mesh 270 may be correlated to a minimum particle size of the therapeutic agent, such that powder is not able to escape into a free space 234 in the reservoir 233 through the plate's mesh size, but the plate 260 still allows some pressurized fluid through the mesh that contributes to powder compaction and reduced aeration. By way of one non-limiting example, if the particles of the therapeutic agent have a diameter of about 75 microns, then the openings in the mesh 270 will be 75 microns or less, thereby avoiding haphazard particle movement in the free space 234.

During use, pressurized fluid flows in a manner generally described above, i.e., from the pressure source 68 of FIGS. 1-2, then from a first end 241 towards a second end 242 of an inlet tube 240. The pressurized fluid flows beneath the platform 235, in a first direction to a second direction, and then is re-directed through an opening 236 in the platform 23 5, where it then flows from a second end 252 towards a first end 251 of an outlet tube 250. As described above with respect to FIGS. 1-3, a combination of the pressurized fluid and the therapeutic agent238 then flows out of the outlet tube 250, and towards the catheter 90 for delivery to the target site.

As therapeutic agent 23 8 is being delivered to the target site, the height of the powder within the container 230 is reduced, and consequently the plate 260 falls in height due to gravity. The provision of the plate 260 improves therapeutic agent flow because the weight of the plate 260 helps to continue compaction of the powder adjacent to the platform 235, and may provide a more predictable dose of powder delivery with each actuation of the button 28 by a user. Further, the plate 260 reduces the ability of the powder to haphazardly compile within the free space 234 of the reservoir 233, which can lead to an inconsistent delivery.

Advantageously, when the plate 260 comprises openings in the mesh 270, the weight and porosity of the plate 260 is designed to prevent over-compression of the powder inside the container 230, but still allows powder aeration. The plate 260 rests atop the powder and allows the pressurized fluid to flow through the powder bed, but without allowing excessive powder dilation (i.e., rising of the powder bed). In short, a plate 260 having mesh 270 can allow some desired movement or shaking of the powder bed, which is desirable, without excessive rising of the powder bed, which may cause drawbacks including inconsistent delivery.

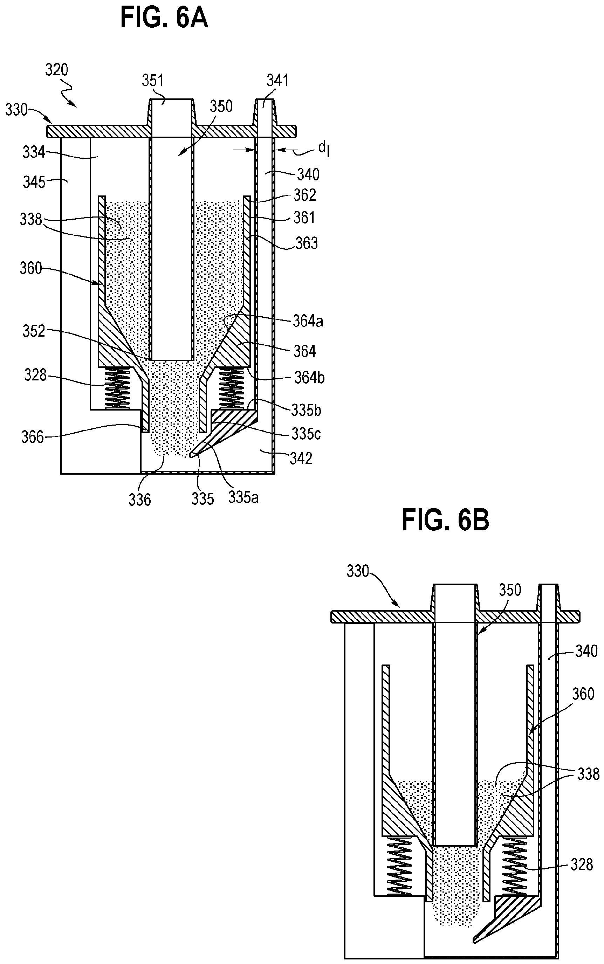

Referring now to FIGS. 6A-6B, a further alternative system 320 comprises a container 330 which is similar to the container 30 of FIGS. 1-3, but which comprises a plate 360 disposed within a reservoir 333 that holds a therapeutic agent 338.

In one embodiment, the container 330 comprises an inlet tube 340 having a first end 341 and a second end 342, which is disposed adjacent to one circumferential side of the container (in the example of FIGS. 6A-6B, the right side in the figures). The inlet tube 340 may protrude into the container 340 by a distance di, as referenced in FIG. 6A. The container 330 may optionally include a blank space 345, i.e., where fluid and therapeutic agent do not flow, at a location opposing the inlet tube 340 (in this example, the blank space 345 is at the left side of the figures).

The plate 360 may be disposed in the container 330 at a circumferential location between the inlet tube 340 and the blank space 345, as shown in FIGS. 6A-6B. In one embodiment, the plate 360 comprises an outer diameter that is substantially the same, or slightly less, than the distance between interior regions of the inlet tube 340 and the blank space 345, as depicted in FIGS. 6A-6B. In this manner, an outer perimeter 361 of the plate 360 is substantially flush with the interior of the container 330, and the plate 360 has an ability to move vertically within the container 330.

In one embodiment, the plate 360 comprises an upper region 362, a central region 364, and a lower region 366. The upper region 362 comprises a vertically upraised segment 363 that is similar to a cylindrical tube, and which comprises the outer perimeter 361 that is substantially flush with the interior of the container 330, as shown in FIGS. 6A-6B.

The central region 364 of the plate 360 may comprise an inward taper 364a and a lower ledge 364b, as shown in FIGS. 6A-6B. The lower ledge 364b of the plate 360 may be positioned above an upper ledge 335b of the platform 335, as depicted in FIGS. 6A-6B.

A resiliently compressible member 328 is disposed vertically between the lower ledge 364b of the plate 360 and the upper ledge 335b of the platform 335, as shown in FIGS. 6A-6B. In one embodiment, the resiliently compressible member 328 may comprise a compression spring, as generally depicted; however, in other embodiments, the resiliently compressible member 328 may comprise other biasing elements, such as compressible foam or other mechanical members which tend to bias the plate 360 upward, as explained below.

The inward taper 364a of the plate 360 transitions into the lower region 366 of the plate 360. The lower region 366 may comprise an upraised segment that is both radially inward of the resiliently compressible member 328, and radially inward of a wall portion 335c of the platform 335, as depicted in FIGS. 6A-6B. In this manner, the plate 360 forms a generally "funnel shape" having the upraised upper region 362, the central tapered region 364, and the upraised lower region 366. The funnel-shaped nature of the plate 360 surrounds the therapeutic agent 338, and provides a tendency to funnel the therapeutic agent 338 towards a tapered section 335a of the platform 335.

During use, pressurized fluid flows in a manner generally described above, i.e., from the pressure source 68 of FIGS. 1-2, then from a first end 341 towards a second end 342 of the inlet tube 340. The pressurized fluid flows beneath the platform 335, in a first direction to a second direction, and then is re-directed through an opening 336 in the platform 335, where it then flows from a second end 352 towards a first end 351 of an outlet tube 350. As described above with respect to FIGS. 1-3, a combination of the pressurized fluid and the therapeutic agent 338 then flows out of the outlet tube 350, and towards the catheter 90 for delivery to the target site.

As therapeutic agent 338 is being delivered to the target site, the plate 360 is free to move in a vertical direction within the container 330, particularly if a user shakes the delivery handle. The provision of the plate 360 improves therapeutic agent flow because the "shaking" movement of the plate 360 helps to continue compaction of the powder adjacent to the platform 335, and may provide a more predictable dose of powder delivery with each actuation of the button 28 by a user. Further, the plate 360 reduces the ability of the powder to haphazardly compile within the reservoir 333, which can lead to an inconsistent delivery.

In one example, the resiliently compressible member 328 provides a desirable vibration effect that helps expedite the settling of powder, while dislodging buildup of stagnant material in the container 330. In short, the ability of the plate 360 to move freely within the container 330, using vibration-assisted action from the resiliently compressible member 328, provides a desirable agitation of the therapeutic agent 338 and may avoid undesirable powder compaction over time.

In the embodiment of FIGS. 6A-6B, it should be noted that, if the resiliently compressible member 328 comprises a spring, then the spring constant may be selected to be a relatively moderate amount. If the sprint constant is too high (rigid), then a desirable vibration may not be imparted to the plate 360. If the spring constant is too low (loose), then too much agitation may occur and it may be difficult to settle down the powder, particularly in-between user-actuated button presses. Thus, a moderate spring constant can be selected to provide a balanced level of movement for the plate 360.

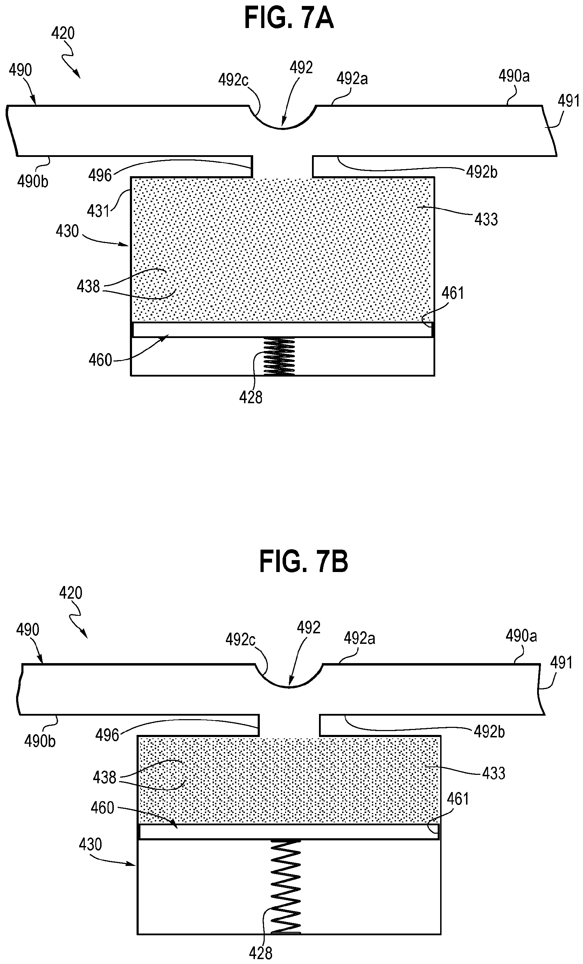

Referring now to FIGS. 7A-7B, a further alternative system 420 comprises a container 430, with a plate 460 disposed within a reservoir 433 that holds a therapeutic agent 438.

In this embodiment, the plate 460 may comprise a circular shape and may be dimensioned such that an outer perimeter 461 of the plate 460 is substantially flush with the interior of the container 430, with the plate 460 having an ability to move vertically within the container 430.

In this example, the therapeutic agent438 is retained above the plate 460 at all times, and further, a resiliently compressible member 428 is disposed vertically beneath the plate 460, as shown in FIGS. 7A-7B. In one embodiment, the resiliently compressible member 428 may comprise a single compression spring that is centered within the container 430, as generally depicted; however, in other embodiments, the resiliently compressible member 428 may comprise multiple compression springs positioned at spaced-apart locations beneath the plate 460, or alternatively may comprise other biasing elements, such as compressible foam or other mechanical members which tend to bias the plate 460 upward, as explained below.

The system of FIGS. 7A-7B further comprises a catheter 490 having an upstream region 490a, a downstream region 490b, and a constriction 492 disposed therebetween. The constriction 492 aligns axially with the container 430, as shown in FIGS. 7A-7B. In one embodiment, a connecting tube 496 is disposed between an upper end 431 of the container 430 and a lower side 492b of the catheter 490, as shown in FIG. 7A, thereby enabling passage of the therapeutic agent 438 from within the container 430 into the catheter 490 via the connecting tube 496.

The constriction 492 may comprises an indented segment 492c formed in the upper side 492a of the catheter 490. The indented segment 492c may extend between about 5% and about 70% into the otherwise unobstructed lumen 491 of the catheter 490. In other words, the constriction 492c may extend between about 5% and about 70% from the upper side 492a towards the lower side 492b of the catheter 490. The constriction 492c may comprise a generally arcuate shape, where the apex of the arc is centered relative to the connecting tube 496 and the container 430, as shown in FIGS. 7A-7B.

Duringuse, pressurized fluid flows from the pressure source 68 of FIGS. 1-2, then from the upstream region 490a towards the downstream region 490b of the catheter 490. The pressurized fluid flows across the constriction 492, at which time a combination of the pressurized fluid and the therapeutic agent 438 then flows out of the container 430 via the connecting tube 496, and towards the downstream region 90b for delivery to the target site. The action of withdrawing the therapeutic agent 43 8 in this manner may also be referred to as a "Venturi effect."

As therapeutic agent 43 8 is being delivered to the target site, then a reduced weight of therapeutic agent 438 is placed upon the plate 460, allowing the plate 460 to move in a vertical direction within the container 430, particularly due to the bias of the resiliently compressible member 428, as shown among the state of FIG. 7A to FIG. 7B.

Advantageously, the resiliently compressible member 428 helps to raise the height of the therapeutic agent438 within the container 430, which facilitates a consistent powder supply to be exposed in the upper region of the container 430 and near the connecting tube 496. The constriction 492 of the catheter 490 at a location vertically aligned with the connecting tube 496 and the container 430 creates the Venturi effect with an increased velocity of the pressurized fluid near the constriction 492, thus drawing the therapeutic agent 438 out of the container 430 and propelling it forward towards the downstream region 490b of the catheter 490. In short, the Venturi-style constriction of the catheter 490 works in synergy with the resiliently compressible member 428 in the container 430, to provide an improved stream of therapeutic agent into the catheter 490.

The present disclosure further includes the following numbered clauses:
Clause 1. A system suitable for delivering a therapeutic agent to a target site, the system comprising: a container for holding the therapeutic agent;
   a pressure source having pressurized fluid, the pressure source in selective fluid communication with at least a portion of the container;
   a catheter in fluid communication with the container and having a lumen sized for delivery of the therapeutic agent to a target site;
   a first inlet tube disposed at least partially within the container, the first inlet tube having a first end that is upstream relative to a second end of the first inlet tube; and
   a second inlet tube disposed at least partially within the container at a location different than the first inlet tube, the second inlet tube having a first end that is upstream relative to a second end of the second inlet tube,
   wherein the pressurized fluid flows into the container via each of the first inlet tube and the second inlet tube.
Clause 2. The system of clause 1, wherein the first and second inlet tubes are spaced-apart in a circumferential direction relative to each other.
Clause 3. The system of clause 2, wherein the first and second inlet tubes are spaced between about 60 degrees and about 300 degrees from one another relative to a circumferential orientation of the container.
Clause 4. The system of clause 1, wherein the second end of the first inlet tube is disposed vertically lower than the second end of the second inlet tube.
Clause 5. The system of clause 1, further comprising a platform positioned within the container, the platform forming a substantially fluid tight seal with an inner surface of the container, wherein the therapeutic agent is retained above the platform.
Clause 6. The system of clause 5, wherein the second end of the first inlet tube is disposed vertically beneath the platform, and the second end of the second inlet tube is disposed vertically above the platform.
Clause 7. The system of clause 6, wherein the second end of the second inlet tube is immersed within the therapeutic agent prior to delivery of the pressurized fluid into the container.
Clause 8. The system of clause 1, further comprising a flow splitter disposed upstream relative to the container, wherein the flow splitter comprises an inlet conduit in fluid communication with the pressure source, and further comprises first and second outlet conduits, wherein the first outlet conduit is in fluid communication with the first end of the first inlet tube, and the second outlet conduit is in fluid communication with the first end of the second inlet tube.
Clause 9. The system of clause 8, wherein the flow splitter evenly distributes flow to the first and second outlet conduits.
Clause 10. The system of clause 8, wherein the flow splitter distributes a higher pressure to the first outlet conduit and into the first inlet tube, wherein the pressure of the fluid delivered through the first outlet conduit is about 1.2 to about 4.0 times greater than the fluid flowing through the second outlet conduit.
Clause 11. The system of clause 1, wherein fluid from the pressure source is directed through a first region of the container in a direction towards a second region of the container, and wherein the fluid is at least partially redirected to urge the therapeutic agent in a direction from the second region of the container towards the first region of the container and subsequently towards the target site.
Clause 12. The system of clause 1 further comprising an outlet tube in fluid communication with a reservoir of the container, wherein the outlet tube is disposed at least partially within the container.
Clause 13. The system of clause 1, wherein the therapeutic agent comprises a powder.
Clause 14. A system suitable for delivering a therapeutic agent to a target site, the system comprising:
   a container for holding the therapeutic agent;
   a pressure source having pressurized fluid, the pressure source in selective fluid communication with at least a portion of the container;
   a catheter in fluid communication with the container and having a lumen sized for delivery of the therapeutic agent to a target site; and
   a plate disposed within the container, wherein the plate is able to move vertically within the container during delivery of the therapeutic agent.
Clause 15. The system of clause 14, wherein the plate comprises a mesh, and wherein the plate is disposed vertically above the therapeutic agent.
Clause 16. The system of clause 15, wherein the mesh comprises openings that are smaller than a size of particles of the therapeutic agent, such that the therapeutic agent remains substantially below the mesh during delivery of the therapeutic agent.
Clause 17. The system of clause 14, further comprising an outlet tube disposed in the container, wherein the plate comprises a first bore, through which the outlet tube extends during use.
Clause 18. The system of clause 17, further comprising an inlet tube disposed in the container, wherein the plate comprises a second bore, through which the inlet tube extends during use.
Clause 19. The system of clause 14, further comprising a resiliently compressible member disposed vertically beneath the plate.
Clause 20. The system of clause 19, wherein the plate comprises an upraised upper region, a tapered central region, and an upraised lower region, wherein the upraised upper region is disposed radially outward compared to the upraised lower region.
Clause 21. The system of clause 19, further comprising a platform positioned within the container, wherein the platform comprises a ledge, and wherein the resiliently compressible member is disposed between the plate and the ledge of the platform.
Clause 22. The system of clause 14, wherein the catheter comprises an upstream region, a downstream region, and a constriction disposed therebetween, wherein the constriction aligns with the container.
Clause 23. The system of clause 22, further comprising a resiliently compressible member disposed vertically beneath the plate, wherein the resiliently compressible member urges the plate towards the constriction as the therapeutic agent is withdrawn from the container.

It will be appreciated in the context of the present disclosure that the present embodiments provide systems and methods suitable for delivering a therapeutic agent to a target site. The system may comprise a container for holding the therapeutic agent, a pressure source, and a catheter in fluid communication with the container. The system may further comprise a first inlet tube disposed at least partially within the container, and a second inlet tube disposed at least partially within the container at a location different than the first inlet tube. Pressurized fluid from the pressure source flows into the container via each of the first inlet tube and the second inlet tube. A plate may be disposed within the container, wherein the plate is able to move vertically within the container during delivery of the therapeutic agent.

While various embodiments of the invention have been described, the invention is not to be restricted except in light of the attached claims and their equivalents. Moreover, the advantages described herein are not necessarily the only advantages of the invention and it is not necessarily expected that every embodiment of the invention will achieve all of the advantages described.

## Claims

1. A system suitable for delivering a therapeutic agent to a target site, the system comprising:
a container for holding the therapeutic agent;
a pressure source having pressurized fluid, the pressure source in selective fluid communication with at least a portion of the container;
a catheter in fluid communication with the container and having a lumen sized for delivery of the therapeutic agent to a target site;
a first inlet tube disposed at least partially within the container, the first inlet tube having a first end that is upstream relative to a second end of the first inlet tube; and
a second inlet tube disposed at least partially within the container at a location different than the first inlet tube, the second inlet tube having a first end that is upstream relative to a second end of the second inlet tube,
wherein the pressurized fluid flows into the container via each of the first inlet tube and the second inlet tube.

2. The system of claim 1, wherein the first and second inlet tubes are spaced-apart in a circumferential direction relative to each other,
for example wherein the first and second inlet tubes are spaced between about 60 degrees and about 300 degrees from one another relative to a circumferential orientation of the container.

3. The system of any preceding claim wherein the second end of the first inlet tube is disposed vertically lower than the second end of the second inlet tube.

4. The system of any preceding claim further comprising a platform positioned within the container, the platform forming a substantially fluid tight seal with an inner surface of the container, wherein the therapeutic agent is retained above the platform
for example, wherein the second end of the first inlet tube is disposed vertically beneath the platform, and the second end of the second inlet tube is disposed vertically above the platform,
for example, wherein the second end of the second inlet tube is immersed within the therapeutic agent prior to delivery of the pressurized fluid into the container.

5. The system of any preceding claim further comprising a flow splitter disposed upstream relative to the container, wherein the flow splitter comprises an inlet conduit in fluid communication with the pressure source, and further comprises first and second outlet conduits, wherein the first outlet conduit is in fluid communication with the first end of the first inlet tube, and the second outlet conduit is in fluid communication with the first end of the second inlet tube,
for example, wherein the flow splitter evenly distributes flow to the first and second outlet conduits,
for example, wherein the flow splitter distributes a higher pressure to the first outlet conduit and into the first inlet tube, wherein the pressure of the fluid delivered through the first outlet conduit is about 1.2 to about 4.0 times greater than the fluid flowing through the second outlet conduit.

6. The system of any preceding claim wherein fluid from the pressure source is directed through a first region of the container in a direction towards a second region of the container, and wherein the fluid is at least partially redirected to urge the therapeutic agent in a direction from the second region of the container towards the first region of the container and subsequently towards the target site.

7. The system of any preceding claim further comprising an outlet tube in fluid communication with a reservoir of the container, wherein the outlet tube is disposed at least partially within the container.

8. The system of any preceding claim wherein the therapeutic agent comprises a powder.

9. A system suitable for delivering a therapeutic agent to a target site, the system comprising:
a container for holding the therapeutic agent;
a pressure source having pressurized fluid, the pressure source in selective fluid communication with at least a portion of the container;
a catheter in fluid communication with the container and having a lumen sized for delivery of the therapeutic agent to a target site; and
a plate disposed with the container, wherein the plate is able to move vertically within the container during delivery of the therapeutic agent.

10. The system of claim 9, wherein the plate comprises a mesh, and wherein the plate is disposed vertically above the therapeutic agent, for example wherein the mesh comprises openings that are smaller than a size of particles of the therapeutic agent, such that the therapeutic agent remains substantially below the mesh during delivery of the therapeutic agent.

11. The system of claim 9 or 10, further comprising an outlet tube disposed in the container, wherein the plate comprises a first bore, through which the outlet tube extends during use, for example wherein the system further comprises an inlet tube disposed in the container, wherein the plate comprises a second bore, through which the inlet tube extends during use.

12. The system of any of claims 9 to 11, further comprising a resiliently compressible member disposed vertically beneath the plate.

13. The system of claim 12, wherein the plate comprises an upraised upper region, a tapered central region, and an upraised lower region, wherein the upraised upper region is disposed radially outward compared to the upraised lower region.

14. The system of claim 12 or 13, further comprising a platform positioned within the container, wherein the platform comprises a ledge, and wherein the resiliently compressible member is disposed between the plate and the ledge of the platform.

15. The system of any of claims 9 to 14, wherein the catheter comprises an upstream region, a downstream region, and a constriction disposed therebetween, wherein the constriction aligns with the container, for example wherein the system further comprises a resiliently compressible member disposed vertically beneath the plate, wherein the resiliently compressible member urges the plate towards the constriction as the therapeutic agent is withdrawn from the container.
